# EUROPEAN PATENT APPLICATION

(11) **EP 3 085 364 A1**
(43) Date of publication of application: **26.10.2016**
(21) Application number: 14872039.4
(22) Date of filing: 18.12.2014
(51) Int. Cl.: A61K 9/28, A61K 9/22, A61K 47/38

(54) **PHARMACEUTICAL COMBINED PREPARATION CONTAINING HMG-COA REDUCTASE INHIBITOR AND CHOLESTEROL ABSORPTION INHIBITOR**

(30) Priority: 18.12.2013 KR 20130158713
(71) Applicant: Alvogen Korea Co., Ltd., Seoul 07326 (KR)
(72) Inventor: LEE, Jae Won, Seoul 156-786 (KR); PARK, Cheol Won, Seoul 120-762 (KR); JUN, Hun, Yongin-si Gyeonggi-do 448-525 (KR); LEE, Tae Won, Suwon-si Gyeonggi-do 443-811 (KR); KIM, Ju Hee, Hwaseong-si Gyeonggi-do 445-826 (KR); CHO, Eun Bi, Suwon-si Gyeonggi-do 442-819 (KR); PARK, Se Hong, Yongin-si Gyeonggi-do 446-987 (KR); LEE, Sun Young, Suwon-si Gyeonggi-do 442-816 (KR)
(74) Representative: Schiweck, Weinzierl & Koch
(86) International application number: PCT/KR2014/012512
(87) International publication number: WO 2015/093859

(57) **Abstract**

The present invention provides a pharmaceutical combination preparation which comprises both of the active ingredients (a HMG-CoA reductase inhibitor and a cholesterol absorption inhibitor) at the same time, and is characterized in that the dissolution pattern of each active ingredient in the combination preparation is the same as that of each active ingredient in the single-ingredient preparations, thereby remarkably improving patient convenience and compliance.

## Description

### TECHNICAL FIELD

The present invention relates to a pharmaceutical combination preparation comprising a HMG-CoA reductase inhibitor and a cholesterol absorption inhibitor.

### BACKGROUND ART

Fats absorbed into the blood - i.e. neutral fat, cholesterol, phospholipid, free fatty acid, etc. - are combined with proteins to form a water-soluble lipoprotein called a serum lipid. A condition where such a serum lipid level is abnormally high, is called dyslipidemia. In order to circulate in the blood, a lipid such as cholesterol, which is insoluble in water, is surrounded by a protein, diffused into the blood and circulated as complexes of lipid and protein called lipoproteins. The lipoproteins for cholesterol-binding and transport are classified into high-density lipoproteins (HDL) and low-density lipoproteins (LDL). HDL removes cholesterol from tissue, and as a result lowers the risk of arteriosclerosis. In contrast, LDL facilitates accumulating cholesterol on the blood vessel wall, thereby increasing the risk of arteriosclerosis.

The biggest cause of arteriosclerosis is the continuous accretion of such unnecessary substances as cholesterol on the blood vessel wall. Among the causes, if the main culprit, LDL, is increased, thereby depositing cholesterol on the blood vessel, then the deposited cholesterol not only narrows the blood vessel, thereby obstructing blood flow, but also advances fibrosis, which causes injury and hardening of the vascular wall. Accordingly, the blood vessel loses its elasticity, facilitating arteriosclerosis, which ultimately results in cardiovascular diseases.

Various methods for treating dyslipidemia are applied, among which cholesterol synthesis inhibitors, 3-hydroxy-3-methylglutaryl-coenzyme A (HMG-CoA) reductase inhibitors, are known as the most effective class of drugs for treating dyslipidemia. The HMG-CoA reductase inhibitors, which are the first-line agents for treating dyslipidemia, may increase the risk of side effects in a dose-dependent manner. Thus, when monotherapy with HMG-CoA reductase inhibitors does not achieve a sufficient therapeutic effect, additional administration of any other drug having a different mechanism of action is preferable than the high-dose treatment with HMC-CoA reductase inhibitors. Furthermore, dyslipidemia may be caused by a combination of several factors such as increases of the total cholesterol, LDL-cholesterol, neutral fat, decreases of HDL-cholesterol, etc. in the blood. Thus, when two or more factors are combined, the combination therapy of several drugs may be considered.

In particular, when a patient requires considerably more decrease in LDL-cholesterol level than in the monotherapy of HMG-CoA reductase inhibitors, combination therapy with ezetimibe is effective. Ezetimibe is a new therapeutic agent for dyslipidemia which selectively inhibits the absorption of cholesterol in the small intestine. Since ezetimibe is different from statins in the mechanism of action, a complementary effect may be expected when it is prescribed together with statins.

Rosuvastatin or its pharmaceutically acceptable salt is [(E)-7-[4-(4-fluorophenyl)-6-isopropyl-2-[methyl(methylsulfonyl)amino]pyrimidin-5-yl]-(3R,5S)-3,5-dihydroxyhept-6-enic acid] (rosuvastatin) and its sodium or calcium salt. They are used specifically in the treatment of hypercholesterolemia and mixed dyslipidemia, and rosuvastatin calcium is sold under the trademark CRESTOR™. Rosuvastatin calcium is an amorphous white or pale yellow powder which is soluble in N,N-dimethylformamide, acetone, or acetonitrile, but is sparingly soluble in water.

Ezetimibe is [1-(4-fluorophenyl)-(3R)-[3-(4-fluorophenyl)-(3S)-hydroxypropyl]-(4S)-(4-hydroxyphenyl)-2-azetidinone)]. It blocks Niemanpick C1-like 1 protein which is known to be involved in cholesterol transportation from the internal organs. It is known that low-density lipoprotein cholesterol (LDL-C) is decreased when ezetimibe is administered, and it is also known that such an effect may be highly enhanced when it is administered in combination with statins. Ezetimibe is sold under the trademark EZETROL™ in the domestic market and ZETIA™ in U.S.A. Ezetimibe is a white powder which is freely to very soluble in ethanol, methanol, or acetone, but is practically insoluble in water. The melting point of ezetimibe is about 163 °C and it is reported to be stable at room temperature.

Further, when monotherapy using statins is less effective for controlling LDL-C, the combination therapy with ezetimibe, which inhibits cholesterol absorption in the small intestine, is recommended as effective. As an example of the combination therapy, VYTORIN™ (simvastatin/ezetimibe) has been developed and marketed (International Patent Application No. PCT/US2003/022889), and Liptruzet™ (atorvastatin/ezetimibe) has been recently approved by the FDA.

Generally, a combination preparation comprising two or more active ingredients may be prepared by using various methods such as designing a formulation that can minimize the decomposition of each active ingredient, using the ingredients combined to minimize unwanted decomposition by-products and to achieve a desirable shelf life, or adding a stabilizer that can improve the stability of active ingredients.

In particular, a combination preparation may comprise a selective excipient that can provide the same dissolution and pharmacokinetic (PK) profile as the control single-ingredient preparation.

### DISCLOSURE

### Technical Problem

Thus, the present inventors have extensively studied to provide a combination preparation which comprises both of the active ingredients of a HMG-CoA reductase inhibitor and a cholesterol absorption inhibitor, specifically rosuvastatin and ezetimibe, which has the same dissolution and PK profile as the control preparation, and at the same time is stable. As a result, the inventors have found that such a combination preparation which is physico-chemically stable and has the same dissolution and PK profile as the control preparation, can be prepared, and have therefore completed the present invention.

### Technical Solution

The present invention is to provide a combination preparation comprising two active ingredients (a HMG-CoA reductase inhibitor and a cholesterol absorption inhibitor) at the same time, wherein the dissolution pattern of each active ingredient in the combination preparation is the same as that of each active ingredient in the single-ingredient preparations.

### Advantageous Effects

The pharmaceutical combination preparation according to the present invention comprises two active ingredients (a HMG-CoA reductase inhibitor and a cholesterol absorption inhibitor) at the same time, and is characterized in that the dissolution pattern of each active ingredient in the combination preparation is the same as that of each active ingredient in the single-ingredient preparations, thereby remarkably improving patient convenience and compliance.

### DESCRIPTION OF DRAWINGS

Figure 1 is a graph showing the dissolution patterns of ezetimibe in the preparations according to the Examples.
Figure 2 is a graph showing the dissolution patterns of ezetimibe in the combination preparations according to the Examples.
Figure 3 is a graph showing the dissolution patterns of rosuvastatin in the combination preparations according to the Examples.

### BEST MODE

The present invention provides a pharmaceutical combination preparation comprising 5 to 15 parts by weight of a HMG-CoA reductase inhibitor; 5 to 15 parts of weight of a cholesterol absorption inhibitor; 4.5 to 12 parts by weight of sodium starch glycolate; and 25 to 35 parts by weight of low substituted hydroxypropyl cellulose.

The term "HMG-CoA reductase inhibitor" as used herein means a drug inhibiting HMG-CoA reductase (3-hydroxy-3-methylglutaryl-coenzyme A reductase) which is an enzyme acting in the step of converting HMG-CoA to mevalonic acid, a rate determining step of cholesterol synthesis. Said HMG-CoA reductase inhibitor has excellent effects of reducing LDL cholesterol and neutral fat, while increasing HDL cholesterol, and is safe and well tolerated with a relatively low frequency of adverse effects. Thus, currently, it is the most widely used in the treatment of dyslipidemia. The HMG-CoA reductase inhibitor may be, for example, any one of simvastatin, rovastatin, atorvastatin, fluvastatin, pravastatin, cerivastatin, pitavastatin, rosuvastatin, and pharmaceutically acceptable salts thereof.

According to the present invention, said HMG-CoA reductase inhibitor is contained in an amount of 5 to 15 parts by weight, preferably 9 to 11 parts by weight in the pharmaceutical combination preparation.

Preferably, said HMG-CoA reductase inhibitor is rosuvastatin or its pharmaceutically acceptable salt, which may be contained in an amount of 5 mg to 50 mg in the pharmaceutical combination preparation.

The term "cholesterol absorption inhibitor" as used herein means a drug inhibiting cholesterol absorption from the small intestine into the circulatory system. Said cholesterol absorption inhibitors may include, for example, ezetimibe or its pharmaceutically acceptable salts. It may be contained in an amount of 5 mg to 50 mg in the pharmaceutical combination preparation.

According to the present invention, said cholesterol absorption inhibitor is contained in an amount of 5 to 15 parts by weight, preferably 9 to 11 parts by weight in the pharmaceutical combination preparation.

The pharmaceutical combination preparation of the present invention comprises both of said active ingredients (a HMG-CoA reductase inhibitor and a cholesterol absorption inhibitor) at the same time, and is characterized in that the dissolution pattern of each active ingredient in the combination preparation is the same as that of each active ingredient in the single-ingredient preparations. Accordingly, the present combination preparation can remarkably improve patient convenience and compliance. Preferably, the pharmaceutical combination preparation of the present invention concurrently comprises rosuvastatin and ezetimibe.

It is important to suitably select the type and amount of excipients in order that the dissolution pattern of each active ingredient in the combination preparation, wherein the combination preparation comprises both of the two active ingredients (a HMG-CoA reductase inhibitor and a cholesterol absorption inhibitor), can be the same as that of each active ingredient in the single-ingredient preparations.

In the present invention, first, the type and amount of excipients are selected and adjusted based on ezetimibe. Specifically, sodium starch glycolate was selected as a suitable excipient in Examples 1 to 5. According to Examples 1 to 5, croscarmellose sodium, sodium starch glycolate, or pregelatinized starch was used as an excipient for ezetimibe, but the use of sodium starch glycolate (Example 3) resulted in the most excellent dissolution, similar to the dissolution pattern of EZETROL™ which is a single-ingredient preparation of ezetimibe.

According to the present invention, said sodium starch glycolate is contained in an amount of 4.5 to 12 parts by weight, preferably 11 to 12 parts by weight in the pharmaceutical combination preparation.

When preparing a combination preparation by adding rosuvastatin to said excipient selected based on said ezetimibe, the dissolution rate of ezetimibe may decline. Specifically, Example 6 shows that the dissolution rate of ezetimibe was somewhat lowered. Without being bound by any theory, the dissolution rate appears to be affected by any interaction between ezetimibe and rosuvastatin.

Thus, in the present invention, the type and amount of excipients are selected for raising the dissolution rate of ezetimibe and at the same time for similarly controlling the dissolution rate of rosuvastatin to that of the single-ingredient preparation.

Specifically, Example 7 shows that the addition of low substituted hydroxypropyl cellulose gave the most excellent dissolution, similar to the dissolution pattern of the single-ingredient preparation of ezetimibe, EZETROL™. Further, the dissolution pattern of rosuvastatin was similar to that of the single-ingredient preparation of rosuvastatin, CRESTOR™.

The term "low substituted hydroxypropyl cellulose" as used herein means the hydroxypropyl cellulose wherein hydroxy groups are partially replaced by hydroxypropoxy groups in a proportion of between 5% to 16%. In the pharmaceutical combination preparation of the present invention, said low substituted hydroxypropyl cellulose is contained in an amount of 30 to 35 parts by weight.

In addition to the above ingredients, the pharmaceutical combination preparation according to the present invention may further comprise calcium hydrogen phosphate in order to increase the stability of said HMG-CoA reductase inhibitor. Said calcium hydrogen phosphate is preferably contained in an amount of 10 to 15 parts by weight. Besides the above, the pharmaceutical combination preparation according to the present invention may further comprise any additives used in the preparation of conventional formulations as long as the additives do not change the efficacy of the pharmaceutical combination preparation. For example, microcrystalline cellulose, magnesium stearate, etc. may be mentioned.

The pharmaceutical combination preparation according to the present invention is characterized in that the HMG-CoA reductase inhibitor has a dissolution rate of 85% or more, preferably 90% or more, more preferably 95% or more within 30 minutes, when measured under the dissolution condition of pH 6.6 buffer solution (900 mL) at 37±0.5°C and at the stirring rate of 50 rpm, according to Method 2 of the Korean Pharmacopoeia dissolution test (Paddle Method).

Further, the pharmaceutical combination preparation according to the present invention is characterized in that the cholesterol absorption inhibitor has a dissolution rate of 80% or more, preferably 85% or more, more preferably 90% or more within 15 minutes, when measured under the dissolution condition of pH 7.0 buffer solution (900 mL; SDS 0.5%) at 37±0.5°C and at the stirring rate of 50 rpm, according to Method 2 of the Korean Pharmacopoeia dissolution test (Paddle Method).

The pharmaceutical combination preparation according to the present invention may be formed from one extrudate comprising the two active ingredients (a HMG-CoA reductase inhibitor and a cholesterol absorption inhibitor); or two separate extrudates each of which comprises one active ingredient; or one extrudate which comprises one or more active ingredients and is subsequently treated with one or more active ingredients which are not provided as an extrudate. Each of said extrudates may further comprise the additives according to the present invention.

The pharmaceutical combination preparation according to the present invention is in the state that the ingredients are uniformly mixed with each other, and may be prepared by methods known in the art such as direct compression, wet granulation, fluid bed granulation, extrusion, solvent evaporation, etc.

Although the total weight of the pharmaceutical combination preparation according to the present invention may differ depending on the prescription, it is preferably in a range of 160 mg to 1000 mg.

Furthermore, the pharmaceutical combination preparation according to the present invention provides a method for treating various disorders due to the elevated level of cholesterol such as dyslipidemia, hyperlipidemia, hypercholesterolemia, atherosclerosis, ateriosclerosis, cardiovascular disease, heart artery disease, coronary artery disease, vascular system disorder and related disorders by administering its therapeutically effective amount to mammals in need thereof according to appropriate dosage regimens.

### MODE FOR INVENTION

Below, preferable examples are provided for assisting the better understanding of the present invention. However, the following examples are provided just for easier understanding of the present invention, but not intended to limit the scope of the present invention.

### Examples 1 to 5: Preparation of ezetimibe granules

For the prescription design of the sparingly soluble ezetimibe, the ezetimibe granules were prepared without rosuvastatin according to the following Examples 1 to 5.

### 1) Example 1

First, a solution was prepared by dissolving 3.5 mg of hydroxypropyl cellulose in 20.0 mg of ethanol. 10.0 mg of ezetimibe, 89.5 mg of lactose hydrate, 26.3 mg of microcrystalline cellulose, 10.9 mg of calcium hydrogen phosphate and 7.5 mg of croscarmellose sodium were mixed together, which was then combined with the above solution to prepare a wet mixture. This wet mixture was passed through #12 to #25 mesh screen and dried in an oven. The dried granule was passed through #16 to #30 mesh screen to prepare a granule mixture. To the resulting granule mixture was added 1.9 mg of magnesium stearate, which was then continuously mixed for 1 to 3 minutes. The composition is shown in Table 1.

### 2) Example 2

The same procedure as Example 1 was carried out except for using 8.0 mg of hydroxypropyl cellulose. The composition is shown in Table 1.

### 3) Example 3

The same procedure as Example 1 was carried out except for using 7.5 mg of sodium starch glycolate. The composition is shown in Table 1.

### 4) Example 4

The same procedure as Example 1 was carried out except for using 7.5 mg of pregelatinized starch. The composition is shown in Table 1.

### 5) Example 5

The same procedure as Example 1 was carried out except for using 3.5 mg of povidone. The composition is shown in Table 1.

**Table 1**

| | | **Example 1** | **Example 2** | **Example 3** | **Example 4** | **Example 5** |
|---|---|---|---|---|---|---|
| **Mixing** | Ezetimibe | 10.0 | 10.0 | 10.0 | 10.0 | 10.0 |
| | Lactose hydrate | 89.5 | 89.5 | 89.5 | 89.5 | 89.5 |
| | Microcrystalline cellulose | 26.3 | 21.8 | 26.3 | 26.3 | 26.3 |
| | Calcium hydrogen phosphate | 10.9 | 10.9 | 10.9 | 10.9 | 10.9 |
| | Croscarmellose sodium | 7.5 | 7.5 | | | 7.5 |
| | Sodium starch glycolate | | | 7.5 | | - |
| | Pregelatinized starch | | | | 7.5 | - |
| **Binder** | Hydroxypropyl cellulose | 3.5 | 8.0 | 3.5 | 3.5 | - |
| | Povidone | | | | | 3.5 |
| | Ethanol | 20.0 | 36.0 | 20.0 | 20.0 | 20.0 |
| **Lubricant** | Magnesium stearate | 1.9 | 1.9 | 1.9 | 1.9 | 1.9 |
| | Total weight | 149.6 | 149.6 | 149.6 | 149.6 | 149.6 |
| All the above values are in mg | | | | | | |

### 6) Dissolution test of ezetimibe

The dissolution test was carried out using the preparations of Examples 1 to 5 under the following conditions. EZETROL™ which is a single-ingredient preparation of ezetimibe was used as the control.

### <Test Condition>

Sample: Examples 1 to 5 and EZETROL™
Dissolution Test Media: pH 7.0 + SDS 0.5%, 900 mℓ, 37±0.5°C
Dissolution Method: Method 2 of the Korean Pharmacopoeia dissolution test (Paddle Method), 50 rpm.

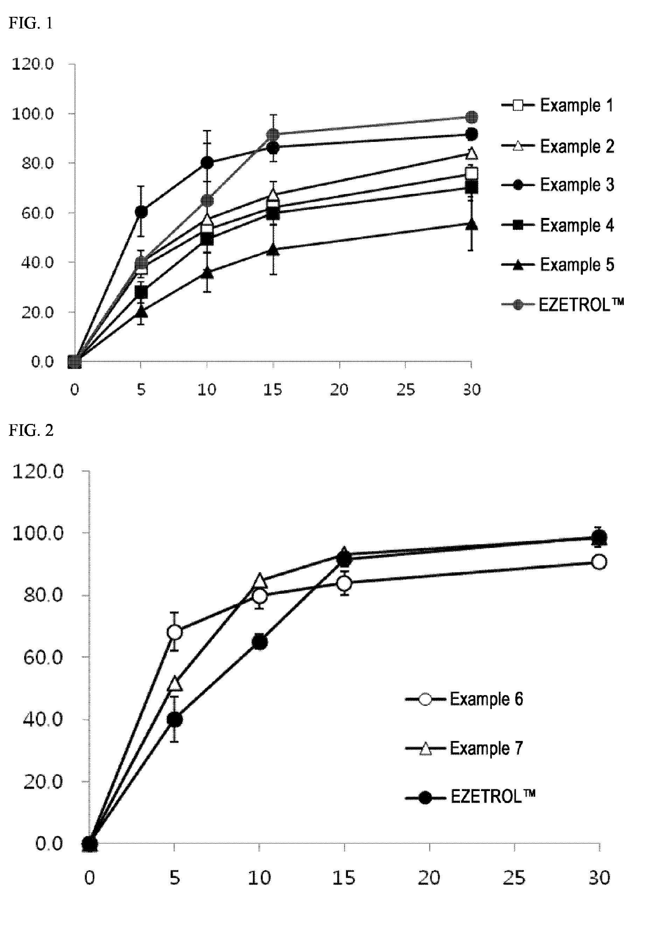

Results of the above ezetimibe dissolution test are shown in Table 2 and Figure 1.

**Table 2**

| | | **0 min** | **5 min** | **10 min** | **15 min** | **30 min** |
|---|---|---|---|---|---|---|
| **Example 1** | Average | 0.0% | 37.8% | 53.1 % | 62.4% | 75.8% |
| | Deviation | 0.0 | 3.8 | 3.4 | 2.5 | 3.6 |
| **Example 2** | Average | 0.0% | 40.2 % | 57.5% | 67.4% | 84.1% |
| | Deviation | 0.0 | 4.7 | 5.7 | 5.1 | 1.2 |
| **Example 3** | Average | 0.0% | 60.6% | 80.3% | 86.4% | 91.7% |
| | Deviation | 0.0 | 10.1 | 7.7 | 5.7 | 2.5 |
| **Example 4** | Average | 0.0% | 28.0% | 49.5% | 60.0% | 70.2 % |
| | Deviation | 0.0 | 4.4 | 5.7 | 5.0 | 5.4 |
| **Example 5** | Average | 0.0% | 20.3% | 36.0% | 45.3% | 55.7% |
| | Deviation | 0.0 | 5.2 | 8.0 | 10.1 | 10.9 |
| **EZETROL™** | Average | 0.0% | 40.1% | 65.0% | 91.6% | 98.7% |
| | Deviation | 0.0 | 0.2 | 27.9 | 7.8 | 0.8 |

As shown in Table 2 and Figure 1, the control group EZETROL™ was fully dissolved after about 30 min, and the Example showing the most similar dissolution pattern thereto was Example 3. Accordingly, the pharmaceutical combination preparation according to the present invention was prepared based on Example 3.

### Examples 6 to 9: Combination preparation of ezetimibe and rosuvastatin

Based on Example 3, the combination preparations further comprising rosuvastatin were prepared as Examples 6 and 7, below.

### 1) Example 6

First, a solution was prepared by dissolving 3.5 mg of hydroxypropyl cellulose in 20.0 mg of ethanol. 10.4 mg of rosuvastatin, 10.0 mg of ezetimibe, 89.5 mg of lactose hydrate, 26.3 mg of microcrystalline cellulose, 10.9 mg of calcium hydrogen phosphate, and 7.5 mg of sodium starch glycolate were mixed together, which was then combined with the above solution to prepare a wet mixture. This wet mixture was passed through #12 to #25 mesh screen and dried in an oven. The dried granule was passed through #16 to #30 mesh screen to prepare a granule mixture. To the resulting granule mixture was added 1.9 mg of magnesium stearate, which was then continuously mixed for 1 to 3 minutes. The composition is shown in Table 3. 4.8 mg of Opadry, a coating agent, per tablet was used for making a coated tablet.

### 2) Example 7

First, a solution was prepared by dissolving 3.5 mg of hydroxypropyl cellulose in 20.0 mg of ethanol. 10.4 mg of rosuvastatin, 10.0 mg of ezetimibe, 79.0 mg of lactose hydrate, 33.0 mg of low substituted hydroxypropyl cellulose, 11.0 mg of calcium hydrogen phosphate, and 11.5 mg of sodium starch glycolate were mixed together, which was then combined with the above solution to prepare a wet mixture. This wet mixture was passed through #12 to #25 mesh screen and dried in an oven. The dried granule was passed through #16 to #30 mesh screen to prepare a granule mixture. To the resulting granule mixture was added 1.6 mg of magnesium stearate, which was then continuously mixed for 1 to 3 minutes. The composition is shown in Table 3. 4.8 mg of Opadry, a coating agent, per tablet was used for making a coated tablet.

### 3) Examples 8 and 9

The same procedure as in Example 7 was carried out except that the ingredients were added in the amounts shown in Table 3.

The compositions of Examples 6 to 9 are shown in Table 3, below. For comparison, Example 3 is also shown therewith.

**Table 3**

| | | **Example 3** | **Example 6** | **Example 7** | **Example 8** | **Example 9** |
|---|---|---|---|---|---|---|
| Mixing | Rosuvastatin | - | 10.4 | 10.4 | 5.2 | 20.8 |
| | Ezetimibe | 10.0 | 10.0 | 10.0 | 10.0 | 10.0 |
| | Lactose hydrate | 89.5 | 89.5 | 79.0 | 84.2 | 168.0 |
| | Microcrystalline cellulose | 26.3 | 26.3 | | - | - |
| | Low substituted hydroxypropyl cellulose | - | - | 33.0 | 33.0 | 66.0 |
| | Calcium hydrogen phosphate | 10.9 | 10.9 | 11.0 | 11.0 | 22.0 |
| | Sodium starch glycolate | 7.5 | 7.5 | 11.5 | 11.5 | 23.0 |
| Binder | Hydroxypropyl cellulose | 3.5 | 3.5 | 3.5 | 3.5 | 7.0 |
| | Ethanol | 20.0 | 20.0 | 20.0 | 20.0 | 40.0 |
| Lubricant | Magnesium stearate | 1.9 | 1.9 | 1.6 | 1.6 | 3.2 |
| | Total weight | 149.6 | 160.0 | 160.0 | 160.0 | 320.0 |
| All the above values are in mg | | | | | | |

### Experimental Example 1: Dissolution Tests

### 1) Dissolution Test for Ezetimibe

The dissolution test was carried out using the preparations of Examples 6 and 7 under the following conditions. EZETROL™ which is a single-ingredient preparation of ezetimibe was used as the control.

### <Test Conditions>

Sample: Examples 6 and 7 and EZETROL™
Dissolution Test Media: pH 7.0 + SDS 0.5%, 900 mℓ, 37±0.5°C
Dissolution Method: Method 2 of the Korean Pharmacopoeia dissolution test (Paddle
Method), 50 rpm.

Results of the above dissolution test for ezetimibe are shown in Table 4 and Figure 2.

**Table 4**

| | | **0 min** | **5 min** | **10 min** | **15 min** | **30 min** |
|---|---|---|---|---|---|---|
| Example 6 | Average | 0.0% | 68.2 % | 79.7% | 83.9% | 90.8% |
| | Deviation | 0.0 | 6.2 | 4.0 | 3.7 | 3.0 |
| Example 7 | Average | 0.0% | 51.6% | 84.7% | 93.2 % | 98.6% |
| | Deviation | 0.0 | 7.2 | 2.5 | 2.4 | 3.1 |
| EZETROL™ | Average | 0.0% | 40.1 % | 65.0% | 91.6% | 98.7% |
| | Deviation | 0.0 | 0.2 | 27.9 | 7.8 | 0.8 |

As shown in Table 4 and Figure 2, the combination preparation of Example 6 wherein rosuvastatin was simply added to Example 3, shows that the dissolution rate of ezetimibe was somewhat lowered (15 min and 30 min). In contrast, the combination preparation of Example 7 wherein the low substituted hydroxypropylcellulose was further added, shows that the dissolution rate of ezetimibe coincided approximately with that of EZETROL™ and the dissolution pattern thereof was almost similar.

### 2) Dissolution Test for Rosuvastatin

The dissolution test was carried out using the preparations of Examples 6 and 7 under the following conditions. CRESTOR™ which is a single-ingredient preparation of rosuvastatin was used as the control.

### <Test Conditions>

Sample: Examples 6 and 7 and CRESTOR™
Dissolution Test Media: pH 6.6, 900 mℓ, 37±0.5°C
Dissolution Method: Method 2 of the Korean Pharmacopoeia dissolution test (Paddle Method), 50 rpm.

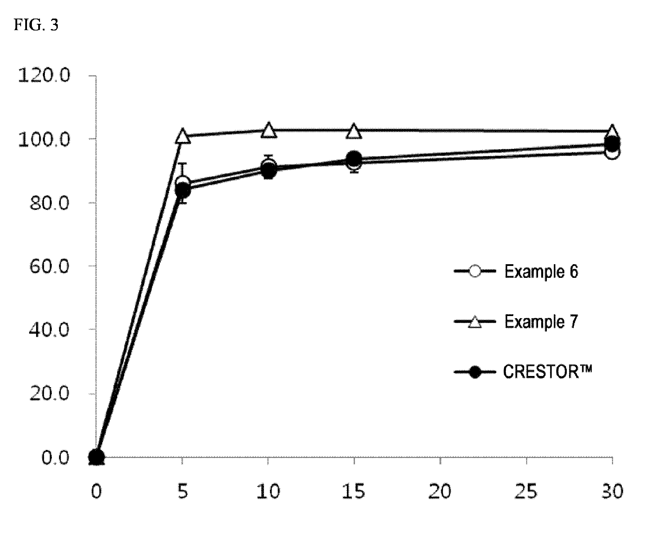

Results of the above dissolution test for rosuvastatin are shown in Table 5 and Figure 3.

**Table 5**

| | | **0 min** | **5 min** | **10 min** | **15 min** | **30 min** |
|---|---|---|---|---|---|---|
| Example 6 | Average | 0.0% | 86.0% | 91.2% | 92.5% | 96.0% |
| | Deviation | 0.0 | 6.2 | 3.6 | 3.0 | 2.3 |
| Example 7 | Average | 0.0% | 100.9% | 102.8% | 102.6 % | 102.3% |
| | Deviation | 0.0 | 1.5 | 1.4 | 1.3 | 1.4 |
| CRESTOR™ | Average | 0.0% | 84.0% | 90.1% | 93.8% | 98.5% |
| | Deviation | 0.0 | 3.6 | 5.1 | 5.5 | 3.6 |

As shown in Table 5 and Figure 3, both of Examples 6 and 7 show a similar dissolution pattern to CRESTOR™, but Example 7 shows a more excellent dissolution.

### Experimental Example 2: In Vivo Pharmacokinetic Tests

In order to compare the *in vivo* pharmacokinetics of the combination preparation of Example 7, rosuvastatin calcium tablet sold as CRESTOR™ (a tablet containing 10 mg of rosuvastatin), and ezetimibe tablet sold as EZETROL™ 10 mg tablet, they were administered under the same conditions to eighteen Beagle dogs (three groups, six dogs per group). The results are shown in Tables 6 and 7, below.

### <Test conditions>

Sample: Combination preparation of Example 7, CRESTOR™ 10 mg tablet, EZETROL™ 10 mg tablet
Individuals per group: Six dogs per group, three groups, a total of 18 dogs
Administration: Single administration of the combination preparation of Example 7 was given to Group 1, CRESTOR™ 10 mg tablet to Group 2, and EZETROL™ 10 mg tablet to Group 3, respectively. The Beagle dogs were randomly distributed among each group.

**Table 6**

| | **AUC (*µ*g.hr/ml)** | **Cmax (*µ*g/ml)** |
|---|---|---|
| CRESTOR™ | 281.95±197.93 | 51.75±19.2 |
| Example 7 | 244.21±109.97 | 63.1±21.5 |

**Table 7**

| | **AUC (*µ*g.hr/ml)** | **Cmax (*µ*g/ml)** |
|---|---|---|
| EZETROL™ | 196.93±128.08 | 22.37±8.63 |
| Example 7 | 161.71±157.76 | 15.9±3.77 |

The pharmacokinetic test results for the combination preparation of Example 7, CRESTOR™ and EZETROL™ were shown in Tables 6 and 7, above. There was no statistically significant difference in the maximum plasma concentration (Cₘₐₓ) between CRESTOR™ and Example 7, and between EZETROL™ and Example 7. Further, there was no statistically significant difference in the area under the curve (AUC). Thus, Example 7 shows equivalent pharmacokinetic profile in the body to each of the controls.

## Claims

1. A pharmaceutical combination preparation comprising
5 to 15 parts by weight of a HMG-CoA reductase inhibitor;
5 to 15 parts by weight of a cholesterol absorption inhibitor;
4.5 to 12 parts by weight of sodium starch glycolate; and
25 to 35 parts by weight of low substituted hydroxypropyl cellulose.

2. The pharmaceutical combination preparation according to Claim 1, wherein said HMG-CoA reductase inhibitor is rosuvastatin, atorvastatin, pitavastatin, or pharmaceutically acceptable salts thereof.

3. The pharmaceutical combination preparation according to Claim 1, wherein said HMG-CoA reductase inhibitor is contained in an amount of 9 to 11 parts by weight.

4. The pharmaceutical combination preparation according to Claim 1, wherein said cholesterol absorption inhibitor is ezetimibe or a pharmaceutically acceptable salt thereof.

5. The pharmaceutical combination preparation according to Claim 1, wherein said cholesterol absorption inhibitor is contained in an amount of 9 to 11 parts by weight.

6. The pharmaceutical combination preparation according to Claim 1, wherein said sodium starch glycolate is contained in an amount of 11 to 12 parts by weight.

7. The pharmaceutical combination preparation according to Claim 1, wherein said low substituted hydroxypropyl cellulose is contained in an amount of 30 to 35 parts by weight.

8. The pharmaceutical combination preparation according to Claim 1, further comprising calcium hydrogen phosphate.

9. The pharmaceutical combination preparation according to Claim 8, wherein said calcium hydrogen phosphate is contained in an amount of 10 to 15 parts by weight.

10. The pharmaceutical combination preparation according to any one of Claims 1 to 9, wherein said HMG-CoA reductase inhibitor has a dissolution rate of 85% or more within 30 minutes, when measured under the dissolution condition of pH 6.6 buffer solution (900 mL) at 37±0.5°C, and at the stirring rate of 50 rpm, according to Method 2 of the Korean Pharmacopoeia dissolution test (Paddle Method).

11. The pharmaceutical combination preparation according to any one of Claims 1 to 9, **characterized in that** said cholesterol absorption inhibitor has a dissolution rate of 80% or more within 15 minutes, when measured under the dissolution condition of pH 7.0 buffer solution (900 mL; 0.5% SDS) at 37±0.5°C and at the stirring rate of 50 rpm, according to Method 2 of the Korean Pharmacopoeia dissolution test (Paddle Method).

12. The pharmaceutical combination preparation according to any one of Claims 1 to 9, wherein the total weight of said pharmaceutical combination preparation is in the range of 160 mg to 1000 mg.
